# FASCICULE DE BREVET EUROPEEN

(11) **EP 1 477 207 B1**
(45) Date de publication et mention de la délivrance du brevet: **25.07.2007**
(21) Numéro de dépôt: 03291023.4
(22) Date de dépôt: 25.04.2003
(51) Int. Cl.: A61N 7/00

(54) **Sonde de therapie**
Therapiesonde
Therapy probe

(30) Priorité: 14.01.2003 FR 0300348
(43) Date de publication de la demande: 17.11.2004
(73) Titulaire: EDAP S.A., 69120 Vaulx en Velin (FR); INSTITUT NATIONAL DE LA SANTE ET DE LA RECHERCHE MEDICALE (INSERM), 75654 Paris Cédex 13 (FR)
(72) Inventeur: Lacoste, François, 75014 Paris (FR); Tetard, Antoine, 69002 Lyon (FR); Chaussy, Christian, Strasslach 82064 (DE); Thueroff, Stephan, 8154 München (DE); Garin, Alexia, 69008 Lyon (FR); Chapelon, Jean-Yves, 69100 Villeurbanne (FR); Lafon, Cyril, 69003 Lyon (FR)
(74) Mandataire: Hirsch & Associés

## Description

La présente invention concerne le traitement des tissus par ultrasons et plus spécifiquement des appareils d'émission d'ultrasons.

La demande de brevet US-A-6 379 320 intitulée " Applicateur intratissulaire ultrasonore pour l'hyperthermie", propose une sonde de coagulation des tissus par des ultrasons thérapeutiques émis par un transducteur plan.

D'autres dispositifs d'émission d'ultrasons sont proposés dans US-A-5 630 837, qui utilise au moins un élément piézoélectrique annulaire ou US-A-5 762 066, dans lequel les éléments piézoélectriques sont disposés dans deux chambres.

Des transducteurs focalisés pour provoquer un échauffement à haute température (technique appelée "HIFU") sont discutés dans FR-A-2 673 542; FR-A-2 700 878; FR-A-2 717 942; FR-A-2 750 340; FR-A-2 778 573; FR-A-2 778 574; FR-A-2 794 018; FR-A-2 807 827; FR-A-2 679 125; FR-A-2 715 822.

Il a aussi été proposé d'utiliser des transducteurs cylindriques dans des sondes - en particulier urétrales - pour produire une émission radiale; US-A 5,391,197 précise que les éléments piézoélectriques sont cylindriques et focalisants. US-A-5,522,869 propose de mesurer la température dans le tissu. US-A-5,549,638 utilise des éléments piézoélectriques cylindriques et mesure la température dans le tissu. US-A-5,620,479 décrit des éléments piézoélectriques tubulaires. US-A-5,733,315 précise que les éléments piézoélectriques sont arrangés autour d'un tube central, certains étant désactivés pour éviter de chauffer le rectum. US-A-5,895,356 précise que les éléments piézoélectriques sont circulaires et focalisants. Ces différents appareils présentent l'inconvénient que le champ ultrasonore est divergent, ce qui peut nuire à l'efficacité en profondeur du traitement.

Il a encore été proposé des transducteurs de thérapie associés à une imagerie de guidage. US-A-5,697,897 décrit un endoscope muni d'une source à ultrasons thérapeutiques. US-A-5,471,988 décrit différentes configurations d'endoscopes munis d'un transducteur de thérapie. Cependant dans tous les cas ce transducteur est focalisant. Le transducteur est associé à un transducteur d'imagerie ou à une optique. US-A-6,050,943 propose des éléments piézoélectriques qui ont 3 fonctions: imagerie, thérapie et contrôle de la température.

Sont encore connues des instruments vibrants, qui comprennent un transducteur couplé à un outil via un conducteur ultrasonore. L'outil peut être une lame ou une pince pour couper ou coaguler les tissus. La coagulation résulte de la montée en température des tissus au contact de l'outil, par friction. La profondeur de coagulation dépend de la conduction thermique du tissu, elle est donc faible. Un instrument appelé "Harmonic Scalpel", scalpel activé par ultrasons est vendu par la société HS ; Ethicon Endo-Surgery, Cincinati, OH, USA.

Divers instruments médicaux utilisent des radiofréquences. Des coagulateurs radio-fréquence utilisent un courant alternatif. On fait passer un courant alternatif dans le tissu qui s'échauffe par effet Ohm. On distingue les coagulateurs bipolaires (l'effet est circonscrit entre les 2 électrodes) et les monopolaires (l'échauffement se produit au voisinage immédiat de la pointe, le courant retourne par une plaque de masse disposée en contact du patient). Des résecteurs endoscopiques disposent tous d'une anse activée par un courant et qui coupe ou coagule les tissus, suivant le courant utilisé. Récemment des anses bipolaires sont apparues. De nombreux autres appareils, identifiés ci-dessous par leur marque, utilisent les radiofréquences :
- Coagulating intermittent cutting (CIC cocut BMP) présente un électrode HF et propose un hachage des périodes de coagulation et des périodes de coupe.
- LigaSure : Pince bipolaire pour sceller les vaisseaux (ESVS Valleylab Boulder, CO USA). Intérêt pour les procédures urologiques : réduction du temps et de la quantité de sang perdue par le patient.

Il a encore été proposé d'utiliser des coagulateurs lasers, avec différents types de laser pour coaguler les veines ou les petits vaisseaux.

Diverses applications des ultrasons dans les traitements sont discutés dans les articles suivants.

| Nom auteur: | titre | Journal |
|---|---|---|
| LAFON C ; CHOSSONS ; PRAT F ; THEILLERE Y ; CHAPELON JY ; BIRER A; CATHIGNOL D | The feasibility of constructing a cylindrical array with a plane rotating beam for interstitial | Ultrasonics, 37(9):615-21 2000 May |
| LAFON C. CHAVRIER F. PRAT F. CHAPELON JY. CATHIGNOL D. | Theorical comparison of two interstitial ultrasound applicators designed to induce cylindrical zones of tissue ablation | Med Biol Eng Comput, 1999, 37:298-303 |
| LAFON C. PRAT F. CHAPELON JY. GORRY F. MARGONARI J. THEILLERE Y. CATHIGNOL D. | In vivo effects of interstitial ultrasound plane applicator Dunning tumours | IEEE, 1998, on 2:1423-1426 |
| LAFON C. PRAT F. CHAPELON JY. GORRY F. MARGONARI J. THEILLERE Y. CATHIGNOL D. | Cylindrical thermal coagulation necrosis using an interstitial applicator with a plane ultrasonic transducer : in vitro and in vivo experiments versus computer simulation | |
| LAFON C. CHAPELON JY. PRAT F. GORRY F. THEILLIERE Y. CATHIGNOL D. | Design and in vitro results of a high intensity ultrasound interstitial applicator | Ultrasonics, 1998, 36: 683-687 |
| LAFON C. CHAPELON JY. PRAT F. GORRY F. MARGONARI J. THEILLIERE Y. CATHIGNOL D, | Design and preliminary results of an ultrasound applicator for interstitial thermal coagulation | Ultrasound in medicine & biology, vol.24, n°1, 113-122, 1998 |
| LAFON C. THEILLERE Y. PRAT F. AREFIEV A. CHAPELON JY. CATHIGNOL D. | Ultrasound interstitial applicator for digestive endoscopy: in vivo destruction of bilary tissues | IEEE, 1999, 2:1447-1450 |
| LAFON C. THEILLERE Y. PRAT F. AREFIEV A. CHAPELON JY. | Development of an interstitial ultrasound applicator for endoscopic procedures: animal experimentation | Ultrasound in medicine & biology, vol.26, n°4, 669-675, 2000 |
| LAFON C; CHAPELON JY; PRAT F; GORRY F; MARGONARI J; THEILLERE Y; CATHIGNOL D | Design and preliminary results of an ultrasound applicator for interstitial thermal interstitial coagulation. | Ultrasound Med Biol, 24(1):113-22 1998 Jan |
| PRAT F. LAFON C. MARGONARI J. GORRY F. THEILLERE Y. CHAPELON JY. CATHIGNOL D. | A high-intensity US probe designed for intraductal destruction: experimental | Gastrointestinal endoscopy, 1999, 50(3): 388-392 results. |
| PRAT F. LAFON C. THEILLERE Y. FRITSCH J. CHOURY AD. LORAND I. CATHIGNOL D. | Destruction of a bile duct carcinoma by intraductal high intensity ultrasound during ERCP. | Gastrointestinal endoscopy, 2001 Jun, 53(7): 797-800 |
| LAFON C.; MELO DE LIMA D.; THEILLERE Y.; PRAT F. CHAPELON JY; CATHIGNOL D. | Optimizing the shape of ultrasound transducers for interstitial thermal ablation | Med. Phys. 29 (3), March 2002. |

Les dispositifs connus posent certains problèmes, qui ne sont pas nécessairement identifiés dans l'état de la technique. Limiter les risques d'hémorragie.

Le chirurgien est toujours confronté au problème de l'hémostase. Il doit coaguler les vaisseaux durant la chirurgie et s'assurer que ceux-ci resteront scellés après l'intervention. Le saignement des artères est souvent facilement identifiable car le sang coule par jet. Les veines sont également problématiques car elles peuvent être difficiles à sceller. Le risque de saignement est particulièrement grave en chirurgie endoscopique puisqu'il est plus difficile à maîtriser. Réduire le risque de réabsorption de glycocolle

La chirurgie endoscopique est souvent faite en milieu aqueux: solution saline ou glycocolle; le glycocolle est le liquide utilisé lors des résections endoscopiques; il est isolant électrique. Quand la pression augmente, il y a réabsorption de glycocolle par le système veineux du patient qui peut conduire à un syndrome appelé *TURP syndrome* en langue anglaise. C'est pourquoi lors des résections endoscopiques (de la prostate, de l'endomètre) on contrôle la quantité d'électrolyte dans le sang et on limite la durée du traitement. Pour ces raisons également, il est important de bien coaguler les veines.

### Limiter le risque de récidives

Lors de la chirurgie carcinologique, l'insertion d'un instrument chirurgical introduit un risque d'essaimage des cellules tumorales dans l'organisme. Dans les cancers de la vessie par exemple, on soupçonne que le simple fait de toucher la tumeur peut accroître le risque de récidive. Il serait donc utile de coaguler les tissus à distance sans les toucher. Etre sélectif

Les instruments traditionnels ont le même effet, que le tissu soit normal ou tumoral. On cherche donc un instrument qui détruirait sélectivement un certain tissu, par exemple tumoral.

Dans certains de ses différents modes de réalisation, l'invention apporte une solution à un ou plusieurs de ces problèmes. D'autres caractéristiques et avantages de l'invention apparaîtront à la lecture de la description détaillée qui suit des modes de réalisation de l'invention, donnés à titre d'exemple uniquement et en références aux dessins qui montrent :
- figure 1, une vue schématique d'un instrument de coagulation endoscopique;
- figure 2, une vue schématique d'un appareil d'endoscopie utilisant l'instrument de la figure 1;
- figures 3 et 4, des vues schématiques d'un appareil de traitement de la prostate;
- figures 5 à 8, des exemples de sondes pour l'appareil des figures 3 et 4;
- figures 9 à 16, des vues schématiques d'instruments de coagulation et de résection;
- figures 17 à 19, des vues schématique d'un coagulateur sectionneur;
- figure 20 à 23; des vues schématique d'un appareil de laparoscopie;
- figures 24 et 25, des graphes des résultats expérimentaux utilisant l'invention.

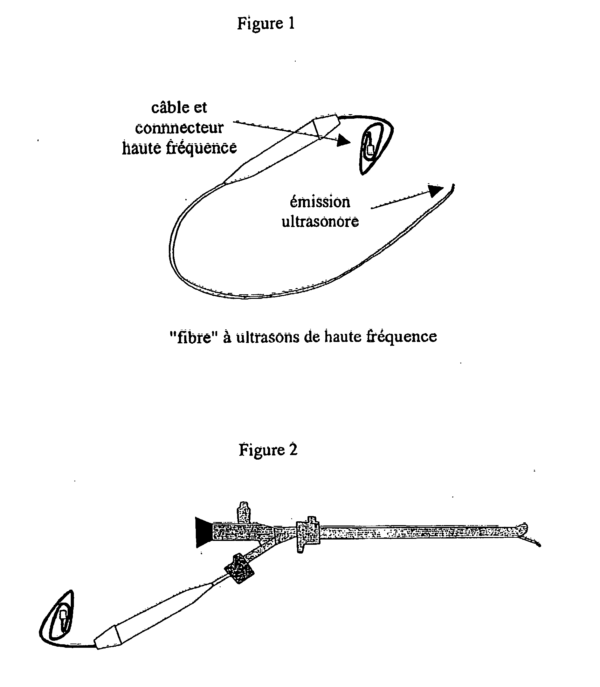

Dans un premier mode de réalisation, l'invention propose d'utiliser un transducteur plan pour la coagulation endoscopique. Le transducteur est utilisé sans membrane, le couplage comme le refroidissement étant assurés par le liquide dans lequel baigne l'organe à traiter. L'instrument présente un câble, au bout duquel est monté le transducteur. Le câble assure l'alimentation du transducteur. Il est souple pour pouvoir être inséré dans des appareils d'endoscopie; s'il assure en même temps une fonction de guidage en translation ou en rotation du transducteur, le câble peut être rigide ou partiellement rigide. La figure 1 montre un schéma de principe d'un exemple de l'instrument: un transducteur est monté à l'extrémité d'un câble souple. L'ensemble est de diamètre assez petit ("fibre") pour passer dans le canal opérateur d'un endoscope, d'une aiguille, d'un cytoscope ou autre, soit de 1 à 5 mm de diamètre. Comme pour la sonde prostatique, le transducteur peut être monté latéralement ou en bout de façon à ce que le champ soit dirigé sensiblement dans l'axe.

Compte tenu des dimensions faibles, on choisira de préférence un transducteur à haute fréquence (8 à 20 MHz), de façon que le champ proche ne diverge pas.

La circulation de liquide de refroidissement et de couplage peut être assurée par les circuits habituellement prévus dans les appareils d'endoscopie. Un circuit d'arrivée débouche à proximité du transducteur; un circuit d'évacuation est aussi prévu, pour aspirer ou recueillir le liquide. Les positions des ouvertures peuvent permettre de diriger le flux de liquide. Le liquide assure à la fois le refroidissement et le couplage ultrasonore.

L'absence de membrane facilite l'insertion et améliore le couplage avec l'organe ou les tissus à traiter, en supprimant une interface et en assurant que le liquide s'étend continûment entre le transducteur et les tissus. On évite aussi toute fenêtre, de sorte que la vision (dans le cas de l'endoscopie) n'est pas limitée; la zone de traitement n'est pas non plus limitée par une fenêtre.

Dans une application de coagulateur à aiguilles ou interstitiel, on utilise l'appareil de la figure 1. A l'inverse de la figure 2, la "fibre" est insérée dans une aiguille. Un tel coagulateur interstitiel peut être utilisé pour le traitement:
- des métastases osseuses
- du foie
- des tumeurs pulmonaires. Particulièrement intéressant parce que les ultrasons étant arrêtés par l'air, on obtient un effet sélectif sur les tumeurs (pleines) alors que le tissu pulmonaire sain (formé d'alvéoles remplies d'air) n'est pas atteint.

La figure 2 montre un exemple de coagulateur dans un appareil d'endoscopie - dans l'exemple un cystoscope. On comprend que la rigidité du câble n'est pas indispensable dans un tel appareil. Les applications cliniques de coagulation endoscopique sont les suivantes:
- tumeurs de la vessie, après résection du polype ou de la tumeur principale. L'appareil sert à stériliser le champ opératoire, afin d'éviter les récidives locales;
- tumeurs de la vessie, pour la coagulation de zones de la muqueuse vésicale repérées par un moyen de visualisation des tumeurs par exemple par fluorescence;
- tumeurs digestives, par exemple polypes rectaux, comme pour la vessie;
- tumeurs pulmonaires, comme décrit plus bas.

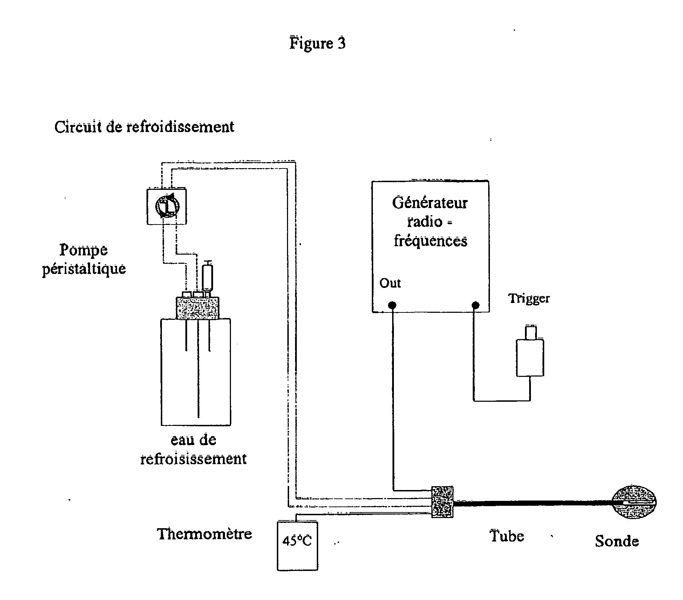

Les figures 3 à 8 montrent un deuxième mode de réalisation. L'appareil est adapté au traitement de la prostate; il comprend une sonde endo-urétrale, avec un transducteur plan et un ballonnet au voisinage de l'extrémité. Le ballonnet permet d'ancrer le dispositif dans la vessie, par gonflage du ballonnet après insertion. Ceci assure un positionnement correct de la sonde, avec le transducteur dans la prostate; optionnellement, le liquide dans le ballonnet peut aussi refroidir, ce qui protège la vessie et le sphincter. On peut comme dans l'exemple des figures 1 et 2 utiliser un transducteur sans membrane; on peut aussi utiliser un circuit distinct pour le gonflage du ballonnet et pour le refroidissement des transducteurs.

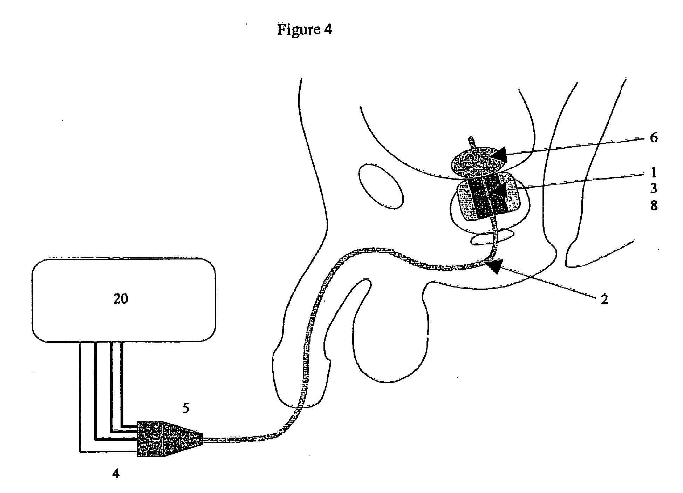

La figure 3 est un schéma de principe de l'ensemble de l'appareil, avec la sonde. On n'a pas représenté le circuit de gonflage du ballonnet. La figure montre notamment la sonde qui apparaît figure 4. La figure 3 montre le coffret sur lequel peut être connectée l'une des sondes décrites dans les figures suivantes.

La figure 4 montre une autre vue schématique de l'appareil. Le coffret 20 comporte l'électronique et un circuit de refroidissement. Divers boutons et voyants sont disposés sur la façade pour le contrôler la puissance ultrasonore à émettre, le débit et la température du circuit de refroidissement. La figure montre le ballonnet 6, le corps 2 de la sonde avec les conduits de liquide de couplage et/ou de refroidissement, connecté en sur le coffret. On reconnaît sur la figure la prostate, avec la zone chauffée. On peut refroidir alternativement ou en combinaison le ballonnet, le ou les transducteurs, ou la paroi de la sonde au niveau des transducteurs ou en amont ou en aval des transducteurs.

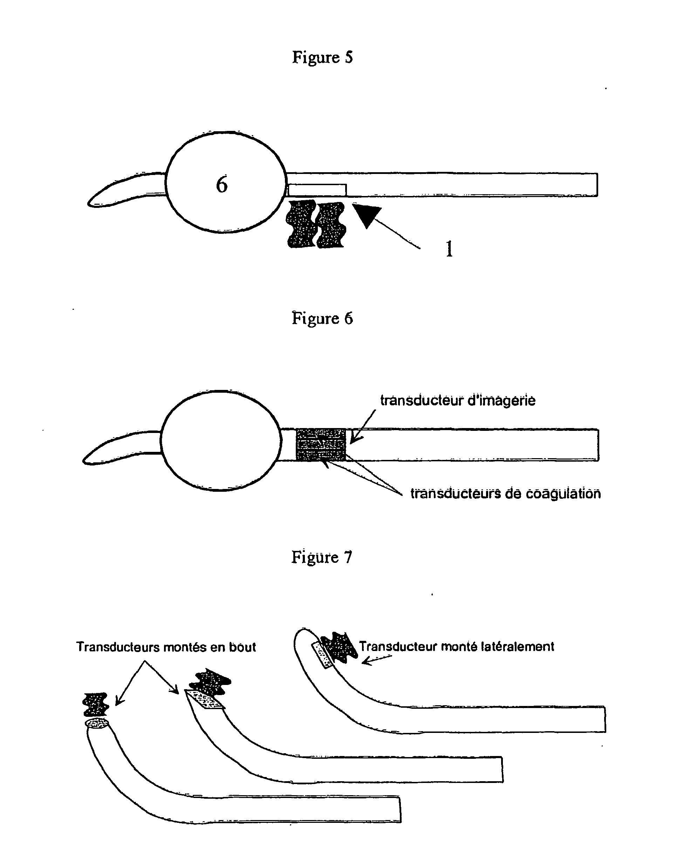

La figure 5 montre la sonde; elle est formée d'un cathéter comportant :
- un transducteur 1 ultrasonore plan ou tel qu'il émet une onde plane collimatée à son extrémité distale,
- des conduits 2 pour le liquide de couplage et de refroidissement,
- optionnellement un capteur 3 de température tel qu'un thermocouple qui permet de s'assurer que le transducteur ne surchauffe pas; on peut aussi mesurer la température locale de la prostate;
- dans sa partie proximale, des connecteurs 4 pour le liquide, l'alimentation électrique du transducteur et la sonde de température;
- optionnellement une interface mécanique, située dans le connecteur assure la rotation de la sonde sur elle-même;
- un ballonnet à son extrémité pour le positionnement de la sonde par rapport au col vésical; l'avantage est que le ballonnet permet de maintenir la sonde en place dans l'urètre,
- optionnellement un fourreau de sonde stérile en matière flexible, dans le cas où le transducteur n'est pas utilisé sans membrane;

Dans l'exemple de la figure 6, on prévoit un transducteur d'imagerie 8, monté en relation mécanique avec le transducteur plan de tir; l'avantage est que l'on visualise les tissus traités. Le transducteur d'imagerie fonctionne à une énergie acoustique largement inférieure à celle du transducteur plan de tir, et n'a pas d'effet notable d'échauffement des tissus. Optionnellement, les fonctions tir et image sont réalisées par un seul et même transducteur, connecté alternativement à une électronique du type "échographe" (fonctionnant en émission impulsionnelle et en réception) ou à un générateur radiofréquence de puissance. On arrive ainsi à réduire l'espace; comme l'image est dans le même plan que la thérapie, on peut contrôler la thérapie de façon précise.

La figure 7 montre que la hauteur et la disposition du transducteur plan peuvent être adaptées à la zone à traiter. Plusieurs modèles de sondes sont proposés:
- suivant la taille de la prostate on peut choisir un transducteur plus ou moins long,
- si on veut traiter les lobes latéraux, on choisira un transducteur à émission latérale, monté latéralement;
- pour le traitement des lobes médians, on choisira un transducteur à émission vers l'avant;
- pour effectuer l'équivalent par voie thermique d'une incision du col vésical, on utilisera un transducteur de faible largeur.

Pour l'utilisation, le chirurgien choisit la sonde la mieux adaptée au patient. La sonde, reliée à son boîtier électronique est introduite dans l'urètre. Le chirurgien peut s'appuyer sur différentes techniques pour guider la sonde: la sonde est mise en place comme n'importe quelle sonde urétrale et maintenue par le ballonnet qui est gonflé dans la vessie, à hauteur du col vésical. Le chirurgien peut vérifier la position de la sonde par échographie transrectale, abdominale ou transurétrale. Dans ce dernier cas, la sonde comportera également un transducteur d'imagerie, par exemple solidaire du transducteur de tir, comme représenté sur la figure 6. Le médecin effectue des tirs vers la prostate. Avantageusement il effectue une vingtaine de tirs en tournant la sonde sur elle-même entre les tirs.

Les suites opératoires sont simples. La coagulation peut être suivie d'une ablation des tissus coagulés, en utilisant un instrument classique ou l'appareil commercialisé sous la marque Rotoresect par Karl Storz. Comme les tissus sont coagulés, l'hémorragie est limitée et l'instrument classique fonctionne de façon plus simple, mieux contrôlée par le chirurgien.

On peut prévoir les variantes suivantes :
- Système à 2 transducteurs dos à dos. 2 transducteurs sont montés dans le même châssis et disposés dos à dos. Ainsi l'émission s'effectue simultanément dans 2 directions opposées, ce qui accélère d'autant le traitement. Par exemple on peut ainsi traiter simultanément les lobes droit et gauche de la prostate.
- Un transducteur émettant des 2 côtés. Le même transducteur peut être utilisé, mettant ainsi à profit les 2 faces d'émission.

De même, on peut disposer des transducteurs multiples, le montage électrique étant en parallèle.

On préfèrera un montage mécanique souple pour introduction dans la prostate. Un transducteur de grande longueur peut être ainsi scindé en plusieurs transducteurs plus courts, alignés le long de l'axe du cathéter, mais monté sur un support flexible, comme discuté plus bas.

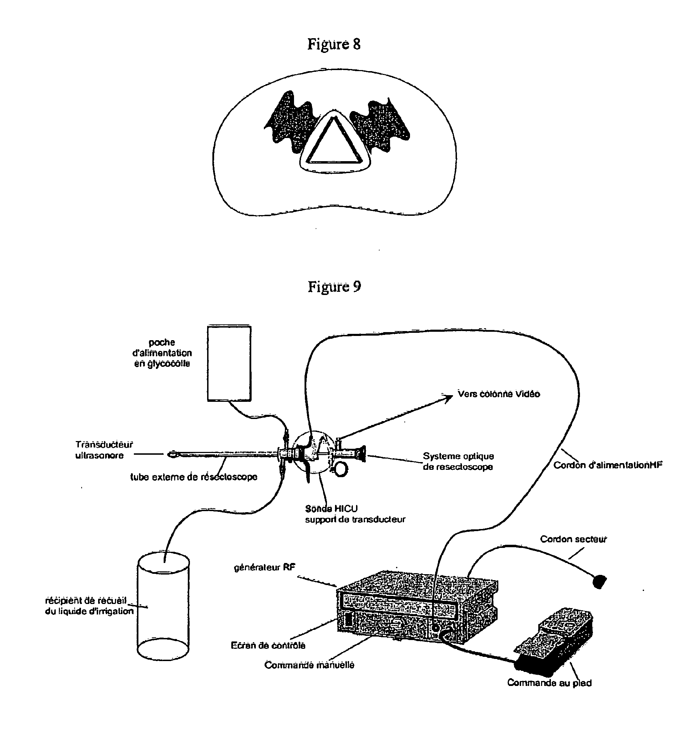

La figure 8 montre un exemple de sonde urétrale profilée. Pour contrôler la position en rotation de la sonde sur elle-même, sa forme peut être non circulaire et épouser l'urètre. Par exemple l'urètre en coupe transversale a souvent une forme triangulaire, la base du triangle se situant en partie postérieure. Avantageusement on peut disposer 2 transducteurs qui émettront l'énergie acoustique en direction des lobes latéraux, comme le montre la figure 8. Si le cathéter porte sonde a une forme semblable, il ne tournera pas lors de l'insertion et le chirurgien sera assuré de l'orientation du transducteur. Le transducteur pourrait aussi tourner à l'intérieur du corps de sonde qui reste fixe. L'avantage est encore un bon contrôle de la position en rotation du transducteur.

Le fourreau de sonde peut être laissé en place après le traitement. Les traitements thermiques des tissus et en particulier de la prostate provoquent généralement un oedème qui fait que le patient se trouve en rétention urinaire après le traitement. Le fourreau est choisi dans un matériau et une épaisseur tels qu'il joue le rôle de prothèse endo-urétrale (stent) et repousse les tissus traités.

L'appareil est adapté au traitement non invasif, non chirurgical de l'adénome de la prostate (en anglais Benign Prostatic Hyperplasia BPH). Par rapport à d'autres traitements non invasifs de la prostate, les avantages sont:
- Faible coût de l'appareil et du consommable
- Rapidité du traitement
- Possibilité de traiter les lobes médians
- Possibilité de simuler des incisions (petites prostates, patient jeunes).

En référence aux figures 9 à 12 est décrit un troisième mode de réalisation de l'invention. Comme dans le premier mode de réalisation, le coagulateur fonctionne sans membrane. Il s'agit d'un appareil de nécrose tissulaire et de coagulation des vaisseaux par ultrasons collimatés (ondes planes) en vue d'une chirurgie non hémorragique.

Cet appareil peut être utilisé :
- par voie urétrale pour le traitement des tissus prostatiques pathologiques; on pourrait aussi prévoir un ballonnet comme décrit ci-dessus;
- en hystérescopie pour la résection endométriale: endomètre pathologique, polypes, fibromes intra utérins;
- En pneumologie pour le traitement des tumeurs pulmonaires. L'avantage dans ce cas est la sélectivité des ultrasons qui vont pénétrer dans les tumeurs, mais pas dans le tissu sain, en raison de la nature alvéolaire de celui-ci

L'appareil peut coaguler le parenchyme et aussi les vaisseaux. L'objectif de cet outil de coagulation est de gagner du temps sur la résection et d'en limiter les risques. La coagulation est plus efficace qu'un résecteur à radio-fréquences parce que l'énergie est apportée plus en profondeur et pendant un temps plus court. Toutes les zones adénomateuses de prostate (ou de l'organe à traiter) sont coagulées directement ou indirectement par la nécrose des vaisseaux les irriguant.

Les bénéfices sont :
- réduction significative des saignements, donc meilleure visibilité du champ opératoire. La longue courbe d'apprentissage de la technique de résection est raccourcie.
- diminution des complications hémorragiques,
- diminution de la quantité de glycocolle absorbée par le patient : le liquide n'est pas en contact avec des vaisseaux béants et le rendement de l'intervention est meilleur (la quantité de tissu enlevée est optimisée par rapport au temps passé car l'hémostase est plus profonde);
- dans le cas de la prostate, amélioration de l'efficacité à long terme de la résection chez les urologues soucieux des complications. En effet, la quantité de copeaux d'adénome enlevée pourra être plus importante que lors d'une procédure classique où le temps passé à l'hémostase n'est pas négligeable;
- gain de temps.

Dans un premier exemple, le dispositif est sans résecteur. Il comprend un applicateur ultrasonore, relié à un boîtier électronique. Le boîtier contient la génération et le contrôle du signal électrique RF de puissance. L'applicateur est destiné à être introduit au contact du tissu à traiter sous contrôle optique. La figure 9 montre un exemple de montage. Comme dans le premier mode de réalisation, le dispositif ne comporte pas de membrane ou de fenêtre transparente aux ultrasons. Le refroidissement du transducteur est assuré par le liquide de rinçage. Le couplage acoustique est également assuré par ce liquide ou, de façon intermittente directement par contact entre le transducteur et le tissu. L'avantage de la suppression de la membrane est une plus grande visibilité du champ opératoire, et une simplicité accrue de l'appareil.

L'applicateur stérile ou stérilisable s'adapte aux résectoscopes. La coagulation se fait sous contrôle de la vue. L'outil coagule le tissu adénomateux et les vaisseaux d'irrigation de la prostate dans la zone de transition. Le passage de la coagulation à la résection et vice versa est très facile et très rapide.

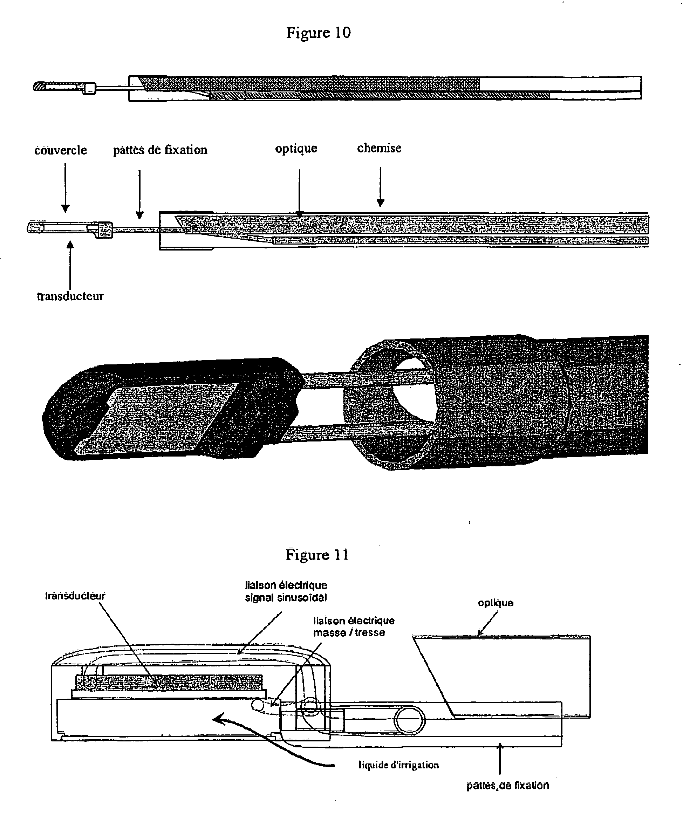

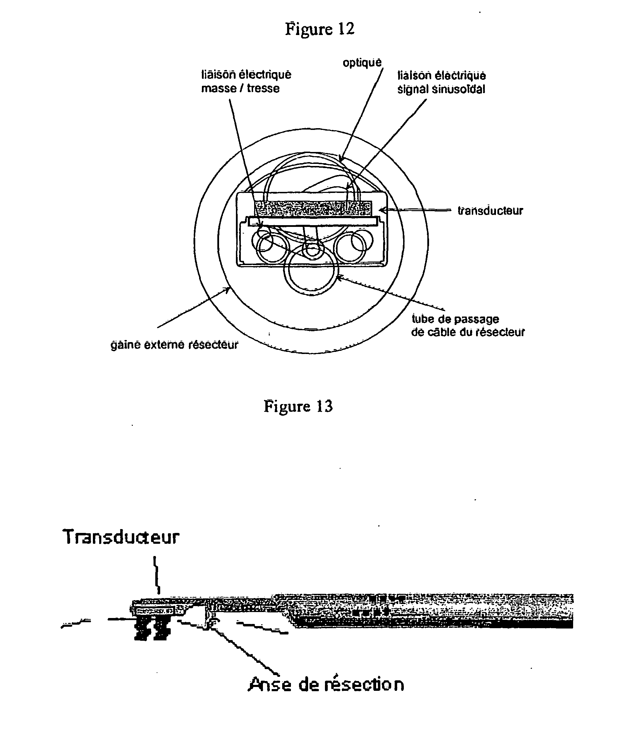

Les figures 10, 11 et 12 montrent le détail de l'applicateur. Le transducteur est monté dans un boîtier, lui-même fixé sur l'optique - ou sur une pièce sur laquelle est fixée l'optique - par des pattes de fixation, dans une disposition telle qu'il n'obscurcit pas le champ. On voit sur la figure 12 que l'ensemble est assez petit pour s'intégrer dans la gaine externe du résecteur. Le liquide d'irrigation peut être dirigé vers le transducteur de manière à le refroidir et assurer le couplage acoustique.

Optionnellement les pattes de fixation sont articulées, de sorte que le faisceau ultrasonore peut être dirigé parallèlement, obliquement ou dans l'axe de l'instrument. Le transducteur peut faire un mouvement de va et vient dans l'axe de l'instrument de la même manière qu'une anse de résection.

Dans un deuxième exemple, le dispositif présente, outre le transducteur plan, une anse de résection. Le chirurgien peut choisir de coaguler le tissu avant ou après la résection. Le transducteur est monté en plus de l'anse de résection; l'avantage est que le même instrument effectue la résection et la coagulation profonde. L'anse de résection coupe et le transducteur coagule.

Le transducteur peut être monté soit:
- solidaire de l'anse de résection et en aval;
- en amont de l'anse
- solidaire du corps du résecteur, auquel cas il reste fixe pendant les mouvements de va et vient de l'anse de résection.

On peut prendre en compte de nombreuses contraintes dans la conception d'un résecteur à coagulation ultrasonore:
- encombrement dans le tube externe du résecteur et à l'avant de l'anse de résection;
- préservation de l'espace réservé à l'irrigation;
- dans le cas d'un dispositif de visualisation, préservation du champ de visibilité du chirurgien;
- le placement du transducteur en avant de l'anse de résection peut également induire des difficultés pour amener l'anse au contact du tissu à enlever (gène mécanique).

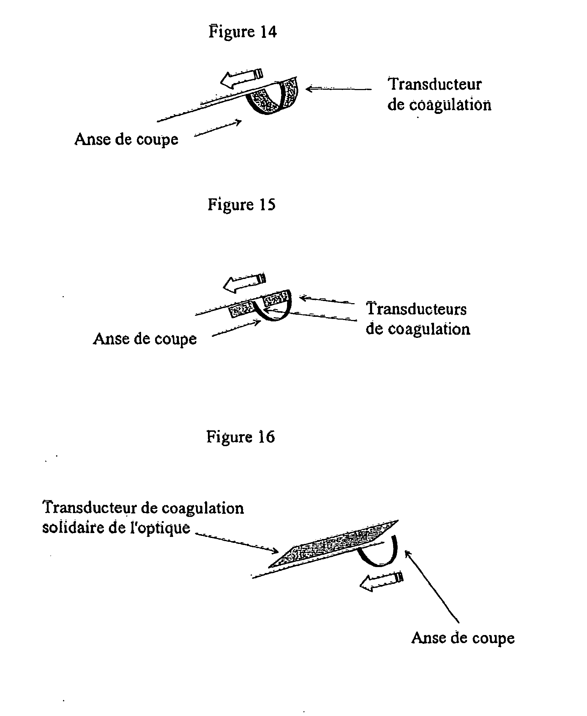

La figure 14 montre une position prenant en compte certaines des ces contraintes; le transducteur est disposé tout au long de l'anse, avec une très faible profondeur de tir, et lame de coupe en bordure du transducteur.

La figure 15 montre un exemple dans lequel le transducteur présente deux éléments attachés aux branches. On place deux transducteurs, par exemple de 2 mm de large et 5 mm de long, de part et d'autre de l'anse fixés sur les branches de maintien afin de libérer le champ médian. On libère ainsi le champ de vision au voisinage de l'anse de résection.

Dans une variante, le transducteur émet vers le bas, le couplage acoustique se fait par le liquide de rinçage, il n'y a donc pas de ballonnet, de membrane ou de fenêtre. Le transducteur est solidaire du corps du résecteur et l'anse est mobile par rapport au transducteur.

Profondeur de tir et vitesse de déplacement. La précision du geste est très importante, par exemple dans le cas de la prostate quand on se rapproche du sphincter. On peut adapter la profondeur de tir sans changer de transducteur, en adaptant le temps de tir et ou la fréquence. La profondeur de coagulation lors du mouvement est réglable entre 1 et 3 mm de préférence sans changer d'outils de coagulation.

Du point de vue du câblage, idéalement le transducteur et le coagulateur sont câblés sur le même réseau électrique de puissance, commandé avec la même pédale, le générateur destiné au coagulateur étant inséré en parallèle sur le générateur habituel, chacun travaillant à sa fréquence et restant peu sensible à la fréquence de l'autre. Un T sur le câble d'alimentation de l'anse de résection permet d'injecter par le même câble le courant d'excitation du transducteur à ultrasons et celui de l'anse. Du côté distal, on peut séparer ces 2 circuits par une électronique appropriée, par exemple des circuits résonnants ou des filtres qui laissent passer uniquement les fréquences de travail de chacun des outils. L'avantage est une réduction de l'encombrement et du câblage.

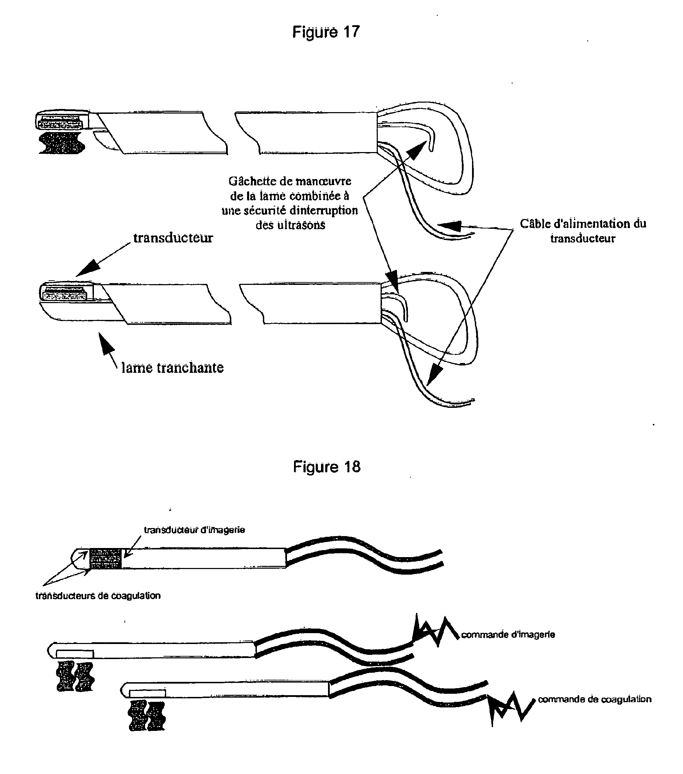

Dans un quatrième mode de réalisation, l'invention propose un coagulateur sectionneur. Il présente un coagulateur ultrasonore associé à une lame de section comme représenté sur la figure 17. Dans un premier temps, le chirurgien coagule le vaisseau au moyen du faisceau ultrasonore. Puis il sectionne le vaisseau en avançant la lame. On peut appuyer sur le vaisseau avec le transducteur pendant la coupe, ce qui évite de devoir maintenir le vaisseau entre 2 pinces. Le transducteur peut fonctionner avec ou sans membrane, selon les applications.

Dans l'exemple de la figure 18, on prévoit aussi un transducteur d'imagerie.

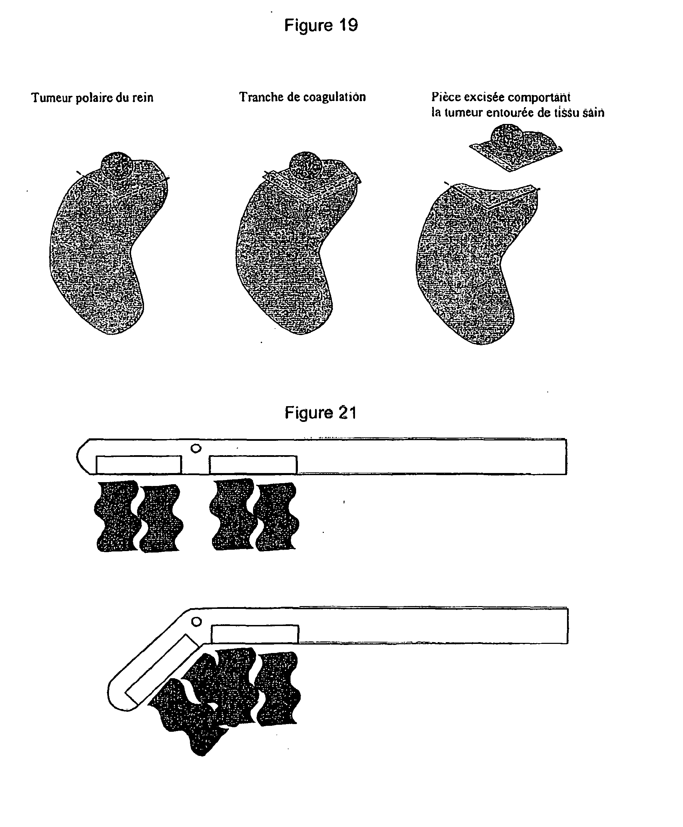

L'objectif de cet outil de coagulation est de pouvoir réaliser plus facilement une néphrectomie partielle, en particulier en laparoscopie. L'intervention d'ablation partielle du rein est aujourd'hui rarement pratiquée en laparoscopie en raison de la très importante vascularisation de l'organe. le risque hémorragique étant important l'intervention laparoscopique est plus dangereuse. L'accessoire de coagulation permet d'effectuer cette intervention sous laparoscopie et limite le temps passé à la suture des vaisseaux. L'applicateur stérile ou stérilisable est inséré dans un trocart. La coagulation se fait sous contrôle de la vue. L'outil coagule le parenchyme rénal et les vaisseaux qui le parcourent sur une tranche, permettant d'isoler la tumeur, comme le montre la figure 19 dans l'exemple d'une tumeur polaire du rein.

La pièce contenant la tumeur est excisée après section sur la tranche de coagulation. L'analyse pathologique habituelle pourra être effectuée à condition d'avoir pris une marge suffisante entre la tumeur et la section du parenchyme rénal. Les bénéfices attendus pour les patients sont ceux de la néphrectomie partielle et ceux de la chirurgie laparoscopique par rapport à l'ablation d'un rein en chirurgie ouverte. Pour un type de chirurgie donnée, les bénéfices sont :
- une réduction de la durée d'intervention,
- une réduction des saignements per-opératoires,

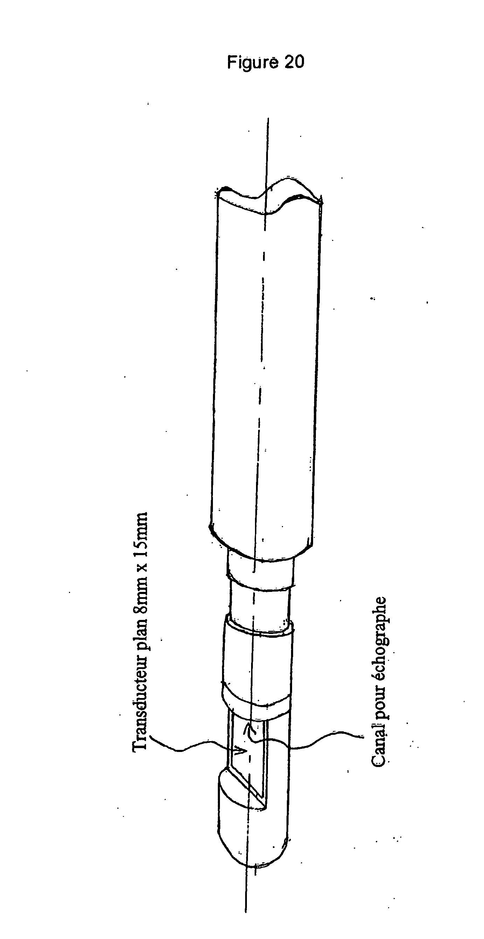

Dans un cinquième mode de réalisation, l'invention propose un appareil de laparoscopie. Elle présente un ou plusieurs transducteurs plan, sur une sonde articulée ou non. La figure 20 montre une sonde avec un transducteur plan d'une taille typique de 8 x 15 mm. Le transducteur s'étend selon le plan médian de la sonde. Le corps de sonde présente un canal pour l'insertion d'une sonde échographique d'angiographie. Ceci permet de visualiser la zone traitée. On pourrait aussi prévoir un tel canal dans l'exemple précédent d'une sonde avec une lame de coupe.

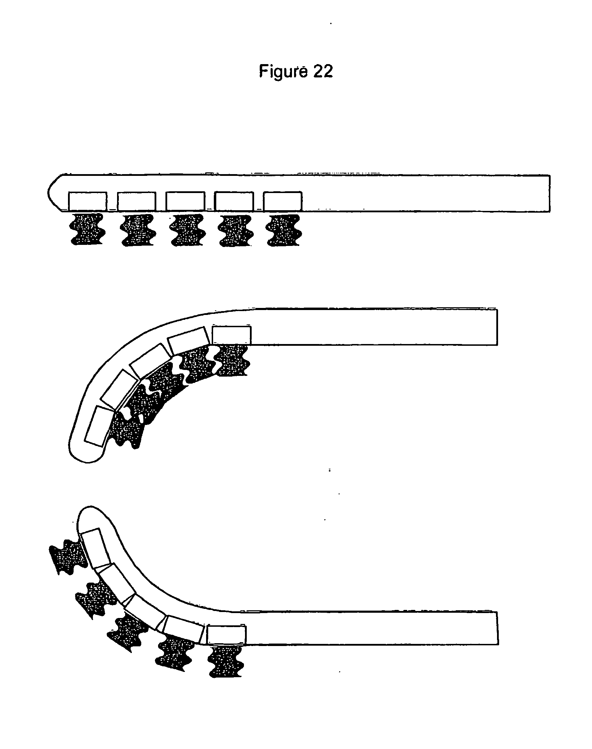

La figure 21 montre des exemples d'une sonde avec plusieurs transducteurs sont montés en ligne le long de la sonde. Eventuellement la sonde est articulée ce qui permet de :
- mieux suivre le pourtour de l'organe - le rein par exemple;
- aux champs ultrasonores de se croiser et ainsi d'augmenter la densité d'énergie au centre du rein; on retrouve un effet de focalisation; toutefois, comme on utilise des transducteurs plans de taille notable, la profondeur de traitement est importante; au centre du rein, on trouve les plus gros vaisseaux sanguins et donc la puissance nécessaire à la coagulation est plus importante qu'en périphérie, ce qui justifie la focalisation.

On entend par taille notable une taille d'au moins 5 mm dans la dimension la plus faible.

La figure 22 montre une autre variante, les transducteurs sont de petite taille et nombreux, de sorte que la sonde est essentiellement souple. On retrouve les avantages cités plus haut. La taille des transducteurs est alors typiquement inférieure à 5 x 8 mm. Avantageusement, on peut commander individuellement la puissance de chacun des transducteurs dans le but de tenir compte de la profondeur à coaguler.

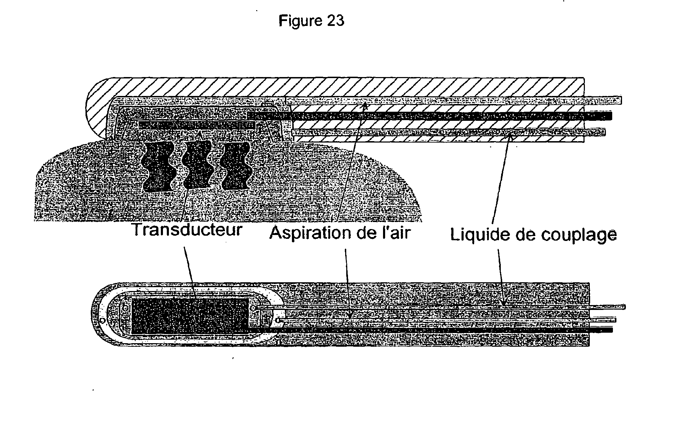

La figure 23 montre encore un autre exemple, dans lequel la sonde est maintenue à la surface du rein par un effet de succion. On prévoit un canal débouchant au voisinage du transducteur, dans lequel on peut faire régner une pression réduite pour fixer en position la sonde par rapport au rein. Le canal est donc adapté à transmettre un vide partiel, en particulier, est suffisamment rigide. Avantageusement, le canal dans lequel on maintient un vide partiel entoure la partie distale de la sonde. Cette variante est combinable avec les variantes décrites plus haut. Par exemple, on peut disposer le canal et le sillon de la figure 23 autour des transducteurs des sondes articulées des figures 21 et 22. L'avantage est que la sonde est maintenue sur l'organe, malgré le déplacement de celui-ci, en raison de la respiration par exemple. La figure montre aussi que l'on peut prévoir un circuit pour le liquide de couplage et de refroidissement. Comme décrit en référence aux figures 1 et 2, on peut ne pas prévoir de membrane ou de fenêtre acoustique devant le transducteur. Ces instruments peuvent aussi être utilisés pour coaguler les tumeurs hépatiques.

Des études précliniques ont été effectuées sur des foies de lapin in vivo avec les paramètres suivants :
- fréquence : 10 MHz ±10%
- puissance acoustique : 14 à 18 W/cm²;
- durée des tirs : 10 à 40 secondes;
- taille du transducteur: 6x10 mm
- nombre de tirs : 5 à 7.

Le pourcentage de coagulation des vaisseaux par rapport à du tissu sain pris comme témoin a été déterminé.

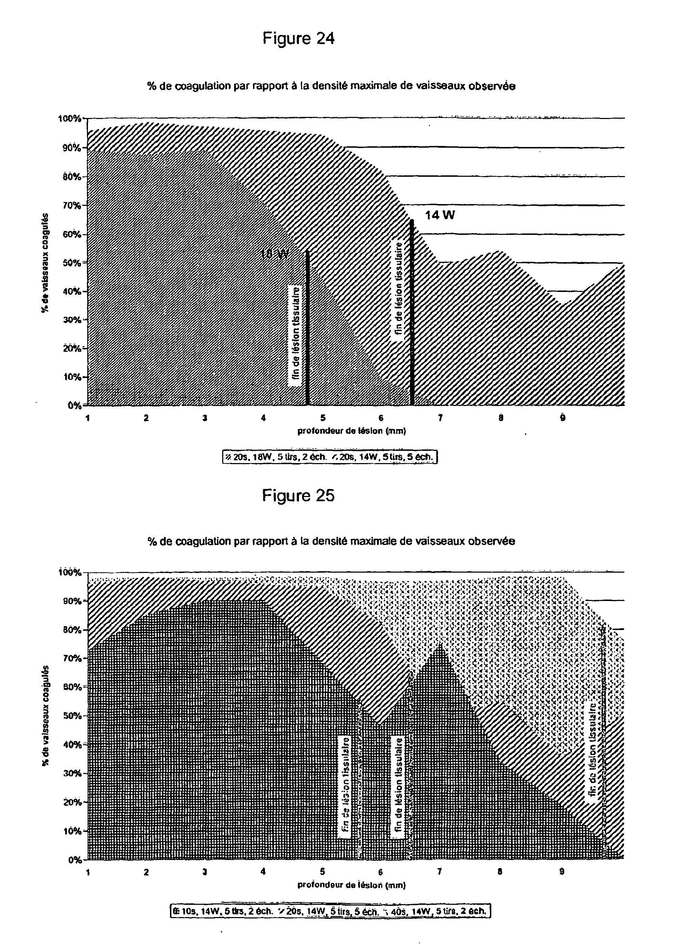

L'influence de la puissance du tir apparaît sur le graphe de la figure 24 où on observe l'influence de la variation de puissance du tir sur la profondeur de coagulation. Les résultats obtenus sont les suivants : contrairement à ce qui était attendu, il est constaté que l'augmentation de la puissance ultrasonore limite la pénétration des ultrasons dans le tissu. Cette diminution de la taille de la lésion ( marqué par l'indicateur de fin de lésion sur les courbes) peut être expliquée par un phénomène de cavitation se produisant lorsque la température du tissu s'élève trop.

Il est également observé que la grande majorité des vaisseaux situés dans la lésion tissulaire est coagulée pour les deux quantités d'énergie acoustique appliquées.

Pour conclure, il n'est pas nécessaire d'augmenter la puissance outre mesure car au-delà d'un certain seuil, le volume total coagulé diminue. On peut donc limiter la profondeur de coagulation en augmentant la puissance.

L'influence de la durée du tir apparaît sur le graphe de la figure 25. La deuxième piste d'exploitation des résultats consiste à observer l'influence de la variation de la durée du tir. Les résultats obtenus sont les suivants : une puissance acoustique de 14 W/cm² a été choisie pour éviter le phénomène de cavitation. Il est constaté que l'augmentation de la durée de l'émission ultrasonore permet d'augmenter la pénétration des ultrasons dans le tissu. Cette augmentation de la taille de la lésion est également liée à la qualité histologique de la coagulation. En effet, lorsque le temps de tir est court (10 secondes), un nombre plus important de vaisseaux situés dans la zone de nécrose tissulaire est épargné, alors que pour des durées de 20 et 40 secondes tous les vaisseaux de la zone de lésion sont obstrués. On peut donc ajuster la profondeur de coagulation en réglant le temps de tir.

Pour conclure, la profondeur de la lésion est intimement liée au temps d'application des ultrasons et il suffit d'apporter une énergie minimale afin d'assurer l'obstruction des vaisseaux, grâce à la forme du transducteur.

Bien entendu, la présente invention n'est pas limitée aux modes de réalisations décrits à titre d'exemple.

## Revendications

1. Un coagulateur ultrasonore présentant un tube de résectoscope, et un transducteur ultrasonore de coagulation à l'extrémité du tube.

2. Le coagulateur de la revendication 1, **caractérisé par** un circuit de fluide de refroidissement et de couplage, avec des ouvertures d'arrivée et d'évacuation du fluide, de préférence au voisinage du transducteur.

3. Le coagulateur de la revendication 1 ou 2, **caractérisé en ce que** le transducteur est monté mobile par rapport au résecteur.

4. Le coagulateur de la revendication 3, **caractérisé en ce que** le transducteur est monté sur des pattes de fixation articulées.

5. Le coagulateur de l'une des revendications 1 à 4, **caractérisé en ce qu**'il comprend en outre une anse de résection.

6. Le coagulateur de l'une des revendications 1 à 5, **caractérisé en ce que** le transducteur est plan.

7. Le coagulateur de la revendication 5, **caractérisé en ce que** le transducteur est conformé à l'anse de résection.

8. Le coagulateur de la revendication 5, 6 ou 7, **caractérisé en ce que** le transducteur est mobile par rapport à l'anse de résection.

9. Le coagulateur de l'une quelconque des revendications 5 à 8, **caractérisé en ce que** le transducteur et l'anse sont alimentés par le même câble.

10. Un appareil de coagulation ultrasonore, présentant un transducteur ultrasonore de coagulation et une lame de coupe mobile par rapport au transducteur.

11. L'appareil de la revendication 10, **caractérisé en ce qu'**il présente en outre un transducteur d'imagerie.

12. Un appareil selon la revendication 10 ou 11, destiné à être utilisé en laparoscopie.

## Claims

1. An ultrasound coagulator having a rectoscope tube with an ultrasound coagulation transducer at an end of said tube.

2. The coagulator according to claim 1, **characterized by** a cooling and coupling fluid circuit with fluid inlet and outlet openings preferably in the region of said transducer.

3. The coagulator according to claim 1 or 2, **characterized in that** the transducer is movably mounted with respect to a resection device.

4. The coagulator according to claim 3, **characterized in that** the transducer is mounted on hinged fastening lugs.

5. The coagulator according to one of claims 1 to 4, **characterized in that** the coagulator further comprises a resection loop.

6. The coagulator according to one of claims 1 to 5, **characterized in that** the transducer is planar.

7. The coagulator according to claim 5, **characterized in that** the transducer has a shape adapted to said resection loop;

8. The coagulator according to claim 5, 6 or 7, **characterized in that** the transducer is movable with respect to said resection loop.

9. The coagulator according to one of claims 5 to 8, **characterized in that** the transducer and the resection loop are powered by the same câble.

10. Ultrasound coagulation apparatus having an ultrasound coagulation transducer and a scalpel blade movable with respect to said transducer.

11. The apparatus of claim 10, **characterized by** further having an imaging transducer.

12. The apparatus of claim 10 or 11, intended for use in laparoscopy.

## Patentansprüche

1. Ultraschallkoagulator, der ein Resektoskoprohr und einen Koagulationsultraschallwandler am Ende des Rohrs aufweist.

2. Koagulator nach Anspruch 1, **gekennzeichnet durch** einen Kreislauf für eine Kühl- und Kopplungsflüssigkeit mit Einlass- und Austrittsöffnungen für die Flüssigkeit, vorzugsweise in der Nähe des Wandlers.

3. Koagulator nach Anspruch 1 oder 2, **dadurch gekennzeichnet, dass** der Wandler im Verhältnis zur Resektionsvorrichtung beweglich befestigt ist.

4. Koagulator nach Anspruch 3, **dadurch gekennzeichnet, dass** der Wandler an beweglich damit verbundenen Halterungen befestigt ist.

5. Koagulator nach einem der Ansprüche 1 bis 4, **dadurch gekennzeichnet, dass** er darüber hinaus eine Resektionsschlinge umfasst.

6. Koagulator nach einem der Ansprüche 1 bis 5, **dadurch gekennzeichnet, dass** der Wandler eben ist.

7. Koagulator nach Anspruch 5, **dadurch gekennzeichnet, dass** der Wandler an die Resektionsschlinge angepasst ist.

8. Koagulator nach Anspruch 5, 6 oder 7, **dadurch gekennzeichnet, dass** der Wandler im Verhältnis zur Resektionsschlinge beweglich ist.

9. Koagulator nach einem der Ansprüche 5 bis 8, **dadurch gekennzeichnet, dass** der Wandler und die Schlinge an dasselbe Kabel angeschlossen sind.

10. Ultraschallkoagulationsgerät, das einen Koagulationsultraschallwandler und eine Schneidklinge aufweist, die im Verhältnis zum Wandler beweglich ist.

11. Gerät nach Anspruch 10, **dadurch gekennzeichnet, dass** es darüber hinaus einen Bildwandler aufweist.

12. Gerät nach Anspruch 10 oder 11, das für die Verwendung bei der Laparoskopie bestimmt ist.
